Europäisches Patentamt

**European Patent Office**

**Office européen des brevets**

(11) Numéro de publication: **0 154 374**

**A2**

(12) **DEMANDE DE BREVET EUROPEEN**

(21) Numéro de dépôt: **85200232.8**

(22) Date de dépôt: **21.02.85**

(51) Int. Cl.⁴: **A 61 K 31/13**
**A 61 K 33/00, A 61 K 33/04**
**A 61 K 31/19, A 23 K 1/16**
**//(A61K33/04, 33:00, 31:19,**
**31:13)**

㉚ Priorité **07.03.84 IT 6721084**

(43) Date de publication de la demande:
**11.09.85 Bulletin 85/37**

(84) Etats contractants désignés:
**BE CH DE FR GB LI LU NL**

⑦ Demandeur **Cagliero, Germano, Dr.**
**Stradale Torino 169**
**I-10015 Ivrea (Torino)(IT)**

(72) Inventeur: **Cirilli, Giovanni**
**Via Fossolo 20/20**
**I-40138 Bologna(IT)**

(72) Inventeur: **Aldana, Clara Susanna**
**Via Selice 47**
**I-40026 Imola (Bologna)(IT)**

(74) Mandataire: **Patrito, Pier Franco, Dr. Ing.**
**Cabinet PATRITO BREVETTI Via Don Minzoni 14**
**I-10121 Torino(IT)**

(54) **Produit pour prévenir et combattre les toxicoses alimentaires provoquées par des mycotoxines.**

㊼ Un produit pour prévenir et combattre les toxicoses alimentaires provoquées par des mycotoxines, lequel contient comme substance active fondamentale de la cystéamine ou un dérivé de celle-ci, et en particulier la 2-mercaptoéthylènediamine. HCL. De préférence, la substance active fondamentale est associée avec au moins un synergiste, constitué par une substance choisie dans le groupe comprenant le carbonate de barium, le propionate de calcium, le diacétate de sodium, le thiosulfate de sodium et le benzoate de sodium, ainsi qu'avec un support. Elle est présentée de préférence sous la forme d'un additif pulvérulent destiné à être ajouté à des denrées alimentaires, et dans ce but on peut avantageusement employer comme support la pectine ou la maltodextrine. Le produit peut être utilisé soit comme additif à joindre aux denrées alimentaires pour prévenir les toxicoses alimentaires provoquées par des mycotoxines, soit comme produit thérapeutique à administrer pour combattre les toxicoses alimentaires provoquées par des mycotoxines. Une formulation préférée du produit est la suivante:

| Support | : Maltodextrine | 700g |
| Substance active | : Cystéamine | 250g |
| Synergiste | : Carbonate de barium | 50g |

PRODUIT POUR PREVENIR ET COMBATTRE LES TOXICOSES ALIMENTAIRES
PROVOQUEES PAR DES MYCOTOXINES

La présente invention se rapporte aux problèmes présentés par les toxicoses alimentaires provoqués par des mycotoxines.

Les toxicoses alimentaires provoquées par des mycotoxines constituent actuellement un problème de portée mondiale. Par l'état actuel des recherches il résulte qu'il y a de nombreux mycètes capables de produire des biotoxines qui peuvent donner lieu à des phénomènes pathologiques parfois létaux. Parmi ces mycotoxines on peut citer particulièrement les aflatoxines, les ocratoxines, le zéralenone et les trychotecènes, qui ont été établis comme les agents potentiels de tumeurs hépatiques, rénales et de l'appareil uro-génital, des syndromes pathologiques bien souvent remarqués dans les Pays dont l'économie est moins avancée.

Par mycotoxines on entend des métabolites secondaires de certains fongus, qui exercent une action biotoxique sur les animaux supérieurs et sur l'homme.

En particulier, les aflatoxines (des dérivés de la coumarine) sont métabolisées par certaines souches de Aspergillus flavus, Aspergillus parasiticus, Aspergillus oryzae et par d'autres mycètes, et elles peuvent être présentes dans tous les aliments "fongueux" ou non (tels que des céréales, graines oléagineuses, fourrages). Biologiquement elles présentent des propriétés cancérogènes, mutagènes et thératogènes.

Dans plusieurs Pays les chercheurs sont à la recherche des facteurs qui peuvent déchaîner la synthèse des mycotoxines, ainsi que de systèmes capables de prévenir cette synthèse. Cependant, les résultats de ces études sont très contrastants, et ils n'ont pas aboutit, jusqu'à maintenant, à des résultats pratiques.

Les méthodes appliquées pratiquement jusqu'ici ont seulement un caractère préventif et elles consistent principalement dans l'emploi d'atmosphères étouffantes (azote et anhydride carbonique) qui ont seulement l'effet de limiter le développement des fongus. D'autres systèmes em-

ployés pour limiter la contamination par des mycotoxines consistent dans la détoxification des denrées alimentaires contaminées, par l'emploi de composés chimiques non spécifiques et parfois potentiellement toxiques ou mutagènes par eux-même, comme l'ammonium, la méthylamine, l'eau oxygénée, l'hydroxyde de calcium, l'hypochlorite de sodium et similaires. Cependant, l'emploi de ces produits non seulement n'a pas résolu le problème, mais dans certains cas il montre une tendance à l'amplifier.

L'emploi de substances antimycotiques pour limiter le développement des fongus peut résulter également nuisible, soit à cause des effets toxiques et mutagènes connus de certaines de ces substances (Pesticide Manual British Corp. Protection Council, edited by Hubert Marton and Charles R. Working, 5. Ed. 1977), soit du fait que certaines de ces substances peuvent hyperstimuler la production de toxines par les fongus dont le développement n'aurait pas été inhibi, en obtenant ainsi un effet opposé à celui qu'on cherchait.

Par ce qu'on a dit ci-haut il résulte qu'il est extrèmement important de connaître les facteurs relatifs à la biosynthèse des mycotoxines afin de pouvoir mettre au point une méthode d'inhibition.

Pour cette raison les auteurs de la présente invention ont effectué avant tout des travaux de recherche dans le but d'établir quels sont les facteurs qui peuvent donner de l'essor à la biosynthèse des mycotoxines.

Le plus grand travail de connaissance et de recherche a été fait pour la plupart sur les aflatoxines, qui sont les mycotoxines les plus toxiques, universellement considérées les plus répandues et qui produisent les plus grands dommages.

Le résultat initial obtenu a été l'observation de la forte production d'aflatoxines sur les graines oléagineuses, une production qui est de loin plus grande par rapport aux graines amylacées.

Pour cette raison on a décidé de commencer une série d'expériences capables d'éclaircir le rôle des lipides en relation avec la biosynthèse des aflatoxines. Pour éclaircir cette problématique on avait décidé initialement d'essayer l'emploi d'inhibiteurs spécifiques de la "fatty a-

cid synthetase", mais le résultat a été d'interférer partiellement dans la croissance des fongus, mais non directement dans la production d'aflatoxines. Cela rendait difficile la corrélation directe de la production des lipides mycéliaires avec la biosynthèse des aflatoxines.

On a pris en considération l'hypothèse que l'effet des inhibiteurs de la "fatty acid synthetase" sur la production des aflatoxines puisse être attribué à la présence de l'anneau époxydique extrêmement réactif. Comme contrôle on a effectué la transformation d'une quote-part des époxydes dans les polyhydroxyacides correspondants. Aussi dans ce cas on a obtenu une forte production d'aflatoxines (environ 100 fois plus grande), mais seulement si dans le terrain de culture était présente la structure époxydique. Aucune stimulation n'a été remarquée lors qu'au lieu des époxydes on introduisait dans le terrain de culture les polyhydroxyacides correspondants.

Les époxydes sont des structures chimiques qui peuvent se former, même si par traces, pendant le rancissement des huiles végétales et animales et des lipides animaux (suif, saindoux et similaires). En plus de ceux-ci se forment des lipoperoxydes, et cela d'autant plus facilement que plus grande est l'insaturation présente dans les lipides, sous l'action de différents agents chimico-physiques (chaleur, oxygène, radiations, catalyseurs minéraux et chimiques, etc.). L'adjonction de lipoperoxydes au terrain de culture a donné lieu à des résultats similaires de ceux obtenus par l'emploi de structures époxydiques. Ce résultat a permis d'expliquer le fait que la production d'aflatoxines est plus grande dans les graines ayant un fort contenu de lipides insaturés.

Puisque la lipoperoxydation est extrêmement influencée par les radicaux libres, on a décidé d'essayer l'effet qui pouvaient exercer sur les mycètes les molécules de méthanes halogénés, connus par leur facilité à mettre à disposition des radicaux libres. On a employé le tétrachlorure de carbone, le chloroforme et le chlorure de méthylène. Avec ces substances aussi, la production d'aflatoxines était considérablement stimulée, d'une façon directement proportionnelle à la facilité avec la-

quelle les différents composés employés produisaient des radicaux libres (le tétrachlorure de carbone stimulait environ 300 fois, le chloroforme environ 150 fois et le chlorure de méthylène environ 100 fois).

Aussi des autres solvants organiques du type du benzène, du cyclohexane, de l'hexane, ont produit des accroissements de la production d'aflatoxines, qui peuvent être rapportés soit à une métabolisation de ces solvants à l'intérieur de la cellule fongine (activation microsomiale avec formation d'intermèdes de nature radicalique), soit à la capacité de ces solvants d'influencer le cours des réactions radicaliques elles-mêmes.

Il a donc été démontré que les époxydes, les lipoperoxydes et les radicaux libres exercent une influence massive sur la production des aflatoxines.

En se basant sur les recherches mentionneés ci-haut on a posé à la base de la présente invention le problème d'inhiber ou de réduire la production d'aflatoxines, ou de bloquer ou ralentir cette production, dans les denrées alimentaires zootechniques, particulièrement dans les céréales, dans les graines oléagineuses, dans les -sous-produits des industries meunières et huilières et de la fermentation, dans les tubercules amilacés et similaires.

Ce but est atteint, selon l'invention, au moyen d'un additif, destiné à être ajouté aux denrées alimentaires, contenant comme substance active fondamentale de la cistéamine ou un dérivé de celle-ci.

En effet, après avoir trouvé les agents principaux qui stimulaient la production des aflatoxines, on a tâché de découvrir parmi les antioxydants connus les réducteurs et les désactiveurs de radicaux qui se révéleraient les plus actifs dans le cas spécifique considéré, et la cystéamine s'est révélée comme la substance la plus apte à bloquer la production des mycotoxines, tout en n'exerçant aucune influence sur la croissance des fongus.

La cystéamine $(HS.CH_2.CH_2.NH_2)$, appelée aussi décarboxycystéine, 2--mercaptoéthylamine, β-amino-éthylmercaptan et amino-éthanthyol, est une substance physiologiquement présente dans les organismes animaux comme produit de décarboxylation de la cystéine. Elle a un caractère basique, et étant peu stable en soi elle est préparée et employée seulement comme chlorhydrate. Ce dernier se présente sous forme de cristaux ayant un point de fusion compris entre 70,2 °C et 70,7 °C, hygroscopiques, très solubles dans l'eau, dans l'éthanol et dans le méthanol, peu solubles dans le chloroforme et dans l'éthylacétate, presque insolubles dans l'éther. Elle est un composant de la pantétheine, qui est à son tour un constituant du coenzyme A.

La cystéamine est connue par son activité thérapeutique contre les dommages à l'organisme dérivant de radiations X; elle agit par un mécanisme d'action qui n'a pas encore été bien eclairci, et elle est efficace si on l'administre avant l'application de la radiation ou même, suivant certains auteurs, après l'application de la radiation. On l'a proposée aussi contre les empoisonnements dûs au thallium et contre les intoxications dues à la streptomycine, et elle est employée exclusivement par voie intraveineuse. La cystéamine est un composé très réactif et on l'utilise aussi comme intermède dans certaines synthèses organiques. Il ne résulte pas qu'elle ait jamais été employée dans la protection des produits alimentaires.

La constatation importante de l'activité de la cystéamine comme inhibiteur de la formation de mycotoxines rend possible et pratique la réduction de la quantité des biotoxines dans les produits alimentaires zootechniques contaminés par des mycètes.

L'invention prévoit d'une façon particulière l'emploi d'un dérivé de la cystéamine, et précisément de la 2-mercaptoéthylènediamine.HCl, qui est une substance produite et commercialisée par la société EGACHEMIE et marquée par le numéro de code 12,292-0. En effet, cette substance s'est révélée comme la plus appropriée pour la réalisation pratique de l'invention.

Une caractéristique ultérieure de l'invention est l'association de la cystéamine avec des substances capable d'expliquer avec elle un sy-

nergisme dans l'action recherchée selon l'invention, et précisément avec au moins une substance choisie dans le groupe comprenant le carbonate de barium, le propionate de calcium, le diacétate de sodium, le thiosulfate de sodium et le benzoate de sodium.

Il s'agit de substances inoffensives et permises par la Loi, dont l'emploi a favorisé d'une façon importante la réalisation de l'objectif préfixé de limiter le développement des fongus et d'inhibir la production des mycotoxines, et en particulier de réduire la quantité d'aflatoxines B, $B_2$, $G_1$, $G_2$, $M_1$ et $M_2$ et d'ocratoxine A, présente dans les produits destinés à l'alimentation zootechnique.

Dans la forme de réalisation préférée de l'invention, les substances actives mentionnées sont employées sous la forme d'un additif pulvérulent destiné à être ajouté aux céréales, aux panneaux, au fourrage ainsi qu'à tout autre produit destiné à l'alimentation animale. Le support pour cet additif pulvérulent peut être constitué par des différentes substances et, particulièrement, par la pectine ou la maltodextrine.

Ci-aprés sont indiqués quelques exemples de formulation de l'additif selon l'invention:

EXEMPLE 1:

| | |
|---|---|
| 2-mercaptoéthylènediamine.HCl | 25 % |
| Carbonate de barium | 5 % |
| Maltodextrine | 70 % |

EXEMPLE 2:

| | |
|---|---|
| 2-mercaptoéthylènediamine.HCl | 25 % |
| Propionate de calcium | 5 % |
| Diacétate de sodium | 5 % |
| Pectine | 65 % |

EXEMPLE 3:

| | |
|---|---|
| 2-mercaptoéthylènediamine.HCl | 25 % |
| Thiosulfate de sodium | 5 % |
| Pectine | 70 % |

0154374

EXEMPLE 4:

| | |
|---|---|
| 2-mercaptoéthylènediamine.HCl | 25 % |
| Benzoate de sodium | 5 % |
| Maltodextrine | 70 % |

PREPARATION: Un additif ayant la composition suivante:

| | | | |
|---|---|---|---|
| Support | : | Maltodextrine | 700 g |
| Substance active | : | Cystéamine | 250 g |
| Synergiste: | : | Carbonate de barium | 50 g |

peut être préparé en ajoutant le synergiste au support, en le dispersant bien, en ajoutant successivement la substance active et en mélangeant bien pendant quelques minutes.

Le produit pulvérulent obtenu selon les exemples précedents est hydrosoluble au 30 %. Il peut être employé en quantité de 50 a 100 g par quintal de fourrage, céréales ou autres aliments, et en ces quantités il élimine le danger de métabolites fongines toxiques ayant une action oncogène, mutagène et thératogène.

Bien que le produit indiqué se soit révélé capable de résoudre le problème technique posé à la base de l'invention, c'est-à-dire le problème de l'inhibition de la formation des mycotoxines dans les produits alimentaires, on a constaté avec surprise que cette action n'est pas sa seule action utile à l'égard du problème général posé par les toxicoses alimentaires dues aux mycotoxines. En effet, le produit a démontré de posséder aussi une activité proprement thérapeutique, dans le sens de provoquer une réversion des lésions produites par les métabolites fongines toxiques. Une telle action thérapeutique ne pouvait d'aucune façon être prévue sur la base de la connaissance des effets thérapeutiques révélés jusqu'à présent par la cystéamine.

Des expériences dans ce sens ont été faites, en particulier, avec des poulets intoxiqués par de l'aflatoxine $B_1$ et par de l'ocratoxine A, avec des lapins intoxiqués par de l'ocratoxine A et par de l'aflatoxine $B_1$, avec des porcs intoxiqués par de l'ocratoxine A, par des aflatoxines $B_1$ et $G_1$ et par de l'aflatoxine $M_1$, et avec des veaux intoxiqués par de

L'aflatoxine $M_1$ et par de l'ocratoxine A.

Les expériences ont été conduites en employant deux procédures différentes:

a) par l'administration du produit pendant 6 jours, dans l'eau à boire, à la concentration de 30 g par hectolitre d'eau;

b) par l'administration pendant 15 jours dans le fourrage, à la concentration de 50 g par quintal de fourrage.

Dans toutes ces expériences on a observé la réversion du syndrome pathologique, le rétablissement des défenses immunitaires, la cicatrisation des lésions provoquées et le retour à la normalité.

On juge que les doses les plus appropriées soient les suivantes: 50 g par quintal de fourrage (dose préventive); 100 g par quintal de fourrage (dose thérapeutique).

Comme il résulte de ce qui précède, l'objet de l'invention est, d'un côté, l'emploi de la cystéamine ou de ses dérivés comme additif pour la protection des denrées alimentaires contre la formation des mycotoxines et, d'autre côté, un nouveau emploi thérapeutique inattendu de la cystéamine ou de ses dérivés pour le traitement des syndromes dérivant de toxicoses alimentaires dues à des mycotoxines.

Par ailleurs, forment objet de l'invention soit le produit spécifique contenant la cystéamine ou un dérivé de celle-ci, ce produit étant destiné à être ajouté aux denrées alimentaires pour prévenir la formation des mycotoxines ou pour conférer aux denrées mêmes un caractère thérapeutique à l'égard des toxicoses alimentaires dues à des mycotoxines, soit les denrées alimentaires qui, grâce à l'adjonction de la cystéamine ou d'un dérivé de celle-ci, ont été rendues résistantes à la formation des mycotoxines ou curatives à l'égard des toxicoses alimentaires dues à des mycotoxines.

Bien que des formulations particulières ont été indiquées à titre d'exemples, la cystéamine ou son dérivé, éventuellement accompagnée par des synergistes, peut être insérée dans n'importe quelle autre formulation appropriée pour l'usage prévu.

REVENDICATIONS

1 . Produit pour prévenir et combattre les toxicoses alimentaires provoquées par des mycotoxines, caractérisé en ce qu'il contient comme substance active fondamentale de la cystéamine ou un dérivé de celle-ci.

2 . Produit selon la revendication 1, caractérisé en ce qu'il a le caractère d'un additif destiné à être ajouté aux denrées alimentaires pour prévenir les toxicoses alimentaires provoquées par des mycotoxines, contenant comme substance active fondamentale de la cystéamine ou un dérivé de celle-ci.

3 . Produit selon la revendication 1, caractérisé en ce qu'il a le caractère d'un produit thérapeutique destiné à être administré afin de combattre les toxicoses alimentaires dues à des mycotoxines, contenant comme substance active fondamentale de la cysteamine ou un dérivé de celle-ci.

4 . Produit selon la revendication 2 ou 3, caractérisé en ce qu'il contient comme substance active fondamentale la 2-mercaptoéthylène-diamine.HCl.

5 . Produit selon la revendication 2, 3 ou 4, caractérisé en ce que la cystéamine ou un dérivé de celle-ci est associé avec au moins un synergiste constitué par une substance choisie dans le groupe comprenant le carbonate de barium, le propionate de calcium, le diacétate de sodium, le thiosulfate de sodium et le benzoate de sodium.

6.- Produit selon l'une quelconque des revendications précédentes, caractérisé en ce que les substances actives mentionnées sont sous la forme d'un additif pulvérulent destiné à être ajouté aux céréales, aux panneaux, au fourrages ou à tout autre produit destiné à l'alimentation animale.

7.- Produit selon la revendication 6, caractérisé en ce que l'additif pulvérulent contient en outre un support, constitué par au moins une substance choisie dans le groupe comprenant la pectine et la maltodextrine.

8.- Produit selon l'une quelconque des revendications précédentes, caractérisé par une formulation proche de l'une des suivantes:

Formulation 1:

| | |
|---|---|
| 2-mercaptoéthylènediamine.HCl | 25 % |
| Carbonate de barium | 5 % |
| Maltodextrine | 70 % |

Formulation 2:

| | |
|---|---|
| 2-mercaptoéthylènediamine.HCl | 25 % |
| Propionate de calcium | 5 % |
| Diacétate de sodium | 5 % |
| Pectine | 65 % |

Formulation 3:

| | |
|---|---|
| 2-mercaptoéthylènediamine.HCl | 25 % |
| Thiosulfate de sodium | 5 % |
| Pectine | 70 % |

Formulation 4:

| | |
|---|---|
| 2-mercaptoéthylènediamine.HCl | 25 % |
| Benzoate de sodium | 5 % |
| Maltodextrine | 70 % |

9. Produit alimentaire, caractérisé en ce qu'il est combiné avec un produit selon l'une quelconque des revendications précédentes afin de le rendre résistant à la formation des mycotoxines ou/et de lui conférer un effet curatif à l'égard des toxicoses alimentaires due à des mycotoxines.